# EUROPEAN PATENT SPECIFICATION

(11) **EP 2 990 415 B1**
(45) Date of publication and mention of the grant of the patent: **28.11.2018**
(21) Application number: 14182795.6
(22) Date of filing: 29.08.2014
(51) Int. Cl.: C07K 14/47

(54) **Cyclic beta defensins analogs for the treatment of infections**
Cyclische Beta-Defensinanaloga zur Behandlung von Infektionen
Analogues de défensines bêta cycliques pour le traitement des infections

(43) Date of publication of application: 02.03.2016
(73) Proprietor: Ceinge Biotecnologie Avanzate S.C. a R.L., 80131 Napoli (IT)
(72) Inventor: COLAVITA, Irene, 80035 Nola (NA) (IT); GALDIERO, Stefania, 80123 Napoli (IT); NIGRO, Ersilia, 81100 Caserta (IT); PESSI, Antonello, 00141 Roma (IT); SALVATORE, Francesco, 80127 Napoli (IT); SCUDIERO, Olga, 80127 Napoli (IT)
(74) Representative: Barchielli, Giovanna

(56) References cited:
- WO-A1-2007/126392
- WO-A2-02/40512
- WO-A2-2009/083249
- WO-A2-2010/092571
- U.S. SUDHEENDRA ET AL: "Membrane disruptive antimicrobial activities of human [beta]-defensin-3 analogs", EUROPEAN JOURNAL OF MEDICINAL CHEMISTRY, vol. 91, 7 August 2014 (2014-08-07), pages 91-99, XP055170363, ISSN: 0223-5234, DOI: 10.1016/j.ejmech.2014.08.021
- NIKOLINKA ANTCHEVA ET AL: "Artificial [beta]-defensin based on a minimal defensin template", BIOCHEMICAL JOURNAL, vol. 283, no. 3, 15 July 2009 (2009-07-15) , pages 5414-447, XP055170562, ISSN: 0264-6021, DOI: 10.1096/fj.07-105015
- HOOVER DAVID M ET AL: "Antimicrobial characterization of human beta-defensin 3 derivatives", ANTIMICROBIAL AGENTS AND CHEMOTHERAPY, AMERICAN SOCIETY FOR MICROBIOLOGY, vol. 47, no. 9, 1 September 2003 (2003-09-01), pages 2804-2809, XP002480114, ISSN: 0066-4804, DOI: 10.1128/AAC.47.9.2804-2809.2003
- OLGA SCUDIERO ET AL: "Novel synthetic, salt-resistant analogs of human beta-defensins 1 and 3 endowed with enhanced antimicrobial activity", ANTIMICROBIAL AGENTS AND CHEMOTHERAPY, AMERICAN SOCIETY FOR MICROBIOLOGY , vol. 54, no. 6 1 June 2010 (2010-06-01), pages 2312-2322, XP002694699, ISSN: 0066-4804, DOI: 10.1128/AAC.01550-09 Retrieved from the Internet: URL:http://aac.asm.org/content/54/6/2312 [retrieved on 2010-03-22]
- DATABASE BIOSIS [Online] BIOSCIENCES INFORMATION SERVICE, PHILADELPHIA, PA, US; November 2013 (2013-11), SCHAJNOVITZ AMIR ET AL: "Human and Murine beta-Defensin-Derived Peptides Induce Rapid Mobilization Of Murine Hematopoietic Stem and Progenitor Cells Via Activation Of CXCR4 Signaling and CXCL12 Release", XP009182676, Database accession no. PREV201400360030

## Description

The present invention refers to cyclic β defensins analogs, to their use in the treatment of infections and pharmaceutical compositions containing them.

It is well known that there is an increasing antibiotic resistance of pathogens and that new strategies are needed to solve this problem. In addition, for some microorganisms, such as *Pseudomonas*, no efficient agents for treatment are even available.

Antimicrobial peptides (AMPs) are considered to potentially provide these necessary new therapeutic tools in the fight against infectious diseases.

AMPs are defence molecules present in and produced by all organisms, from bacteria to vertebrates. These peptides exert multiple effective activities against all microorganisms, including bacteria, fungi, viruses, and parasites. In humans and other mammals AMPs are a key component of the epithelial surface defence, constituting an impediment between the outer and the inner environment, interacting with diverse microrganisms.

Defensins are AMPs showing a characteristic amphipathic nature derived from positive charges and hydrophobic amino acid residues which are organized to assume a three dimensional amphiphatic structure. Defensins have been classified in three subfamilies: alpha, beta and teta defensins. Six cysteine residues responsible for three intramolecular disulfide bonds combined in different pattern are recognized features of these peptides. The folding of defensins is mainly stabilized by the presence of these three disulphide bonds which determine the distribution of positively charged and hydrophobic residues which is correlated to their activity.

Defensins are able to disrupt the membrane structure of microorganisms; moreover, they can inhibit the synthesis of the DNA and affect the metabolism turnover. Several authors demonstrated a wide spectrum antimicrobial activity versus several microorganisms, like Gram-positive and Gram-negative bacteria, fungi, and viruses.

For these reasons, defensins are high-quality candidates, able to fight infectious diseases.

Defensins have also a number of disadvantages that severely restrict their clinical use. For example, they can exert hemolytic activity (6), their activity can be inhibited in presence of high salt concentration (7) and they are rapidly cleaved in human fluids by proteases. The correct folding of the disulphide bonds also represents a limiting factor to the potential therapeutic use of defensins. In fact, the obtainment of a single molecule with the correct pattern of disulfide bonds is extremely complicated and expensive.

In an attempt to overcome these disadvantages, chimeric β-Defensins combining human β-defensin 3 (hBD3) with either human β-defensin 1 (hBD1) (WO2009/083249; Scudiero O. et al. Antimicrob. Agents Chemother. 2010; 54: 2312-2322; Scudiero O. et al. Antimicrob. Ag. Chemother. 2013; 57: 1701-1708;) or human β-defensin 2 (hBD2) (Jung S. et al. Antimicrob. Agents Chemother. 2011; 55: 954-960; Spudy B. et al. Biochem. Biophys. Res. Commun. 2012; 427: 207-211) have been synthetized and investigated for their antibacterial, antiviral and chemotactic activities, as well as their salt resistance. The synthetic analogs have shown increased antibacterial properties, and reduced salt sensitivity.

Despite optimization of the synthetic protocols (Liu S.. Pept. Sci. 2009; 15: 95-106) the preparation of hDB3 and analogs is very challenging, and does not lend itself easily to large-scale commercial production. Moreover, suitable pharmacokinetics and biodistribution has not yet been shown for any β-Defensin therapeutic candidate.

We have now identified cyclic β defensins analogs with a more favourable profile in terms of ease of synthesis, cost-of-goods, in vitro and in vivo stability, which maintain as much as possible the biological profile of full-length hBD3.

The cyclic β defensins analogs of the present invention maintain the C-terminal tail of hBD3 and comprise segments which are part of the aminoacidic sequences of the ring closed by the native disulfide Cys³-Cys⁶ in the human β-Defensins hBD1-hBD3.

Object of the present invention is a cyclic peptide selected from the group consisting of SEQ. ID. No. 1, SEQ. ID. No. 2, SEQ. ID. No. 25.
SEQ. ID. No. 1 corresponds to formula (I) : 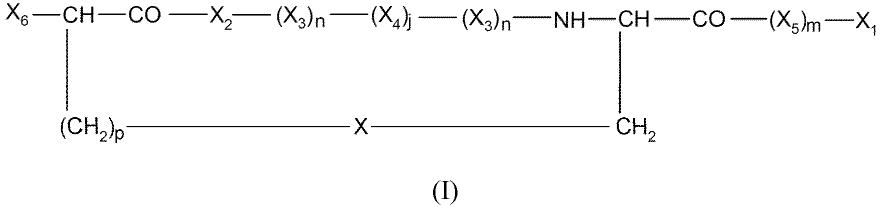 wherein:
   X is -S-S-;
   X₁ is RRKK wherein the amino acid residues have L-configuration;
   X₂ is LPKEEQIGK;
   X₃ is Ser and n is 1;
   X₄ is STRGRK and j is 1;
   m is 0;
   X₆ is CH₃-CO-NH- and p is 1.
SEQ. ID. No. 2 corresponds to formula (I) wherein:
   X is -S-S-;
   X₁ is RRKK wherein the amino acid residues have L-configuration;
   X₂ is LPKQQQIGK;
   X₃ is Ser and n is 1;
   X₄ is STRGRK and j is 1;
   m is 0;
   X₆ is CH3-CO-NH- and p is 1.
SEQ. ID. No. 25 corresponds to formula (I) wherein:
   X is -S-S-;
   X₁ is the sequence RRKK wherein the amino acid residues have L-configuration;
   X₂ is PIFTKIQGT;
   n is 0;
   j is 0;
   X₅ is GG and m is 1;
   X₆ is NH₂- and p is 1.

The β defensins analogs of the present invention can be administered, if desired, in the form of pharmaceutically acceptable salts. Salts may be prepared using standard procedures known to those skilled in the art of synthetic organic chemistry.

The β defensins analogs of the present invention can be lipidated (either conjugated with cholesterol or fatty acids, such as palmitoylacid) or PEGylated in order to improve in vivo pharmacological profile. The method for obtaining these derivatives are well known in the art.

The β defensins analog of formula (I) as above defined can be used as antibacterial and/or antiviral agent in particular they can be used in the treatment of infections such as infection by herpes simplex virus, human immunodeficiency virus, hepatitis C virus, influenza virus, cytomegalovirus, human papilloma virus, human parainfluenza virus, respiratory syncytial virus, varicella zooster virus, and vaccinia virus (see Wilson, S. S., M. E. Wiens, et al. (2013). "Antiviral mechanisms of human defensins." J. Mol. Biol. 425(24): 4965-4980).

Another object of the present invention are pharmaceutical compositions comprising a therapeutically-effective amount of a β defensins analog of formula (I) as above defined and at least one pharmaceutically acceptable excipient and/or carrier. Such a composition may also be comprised of diluents, fillers, salts, buffers, stabilizers, solubilizers, and other materials well known in the art.

The pharmaceutical composition may be in any suitable form, depending on the intended. method of administration. It may for example be in the form of a tablet, capsule or liquid for oral administration, or of a solution or suspension for administration parenterally.

The dosage rate at which the compound, derivative or composition is administered will depend on the nature of the subject, the nature and severity of the condition, the administration method used, etc. Appropriate values can be selected by the trained medical practitioner.

The compounds of the invention may be administered by any suitable route, including orally, intravenously, cutaneously, subcutaneously, etc. These compounds may be prepared by chemical synthesis beginning from individual, preferably protected, amino acids or oligopeptides and using known peptide synthesis methods such as the standard conventional room temperature 9-fluorenylmethoxycarbonyl (Fmoc) chemistry as described in Atherton, E. and R. C. Sheppard (1989). Solid-phase peptide synthesis, a practical approach. Oxford, IRL Press.

### EXAMPLES

### Example 1: Synthesis of peptides

### Reagents

All Fmoc amino acids, Diisopropylethylamine (DIPEA), piperidine, Acetic anhydride, trifluoroacetic acid (TFA), triisopropylsilane (TIS), Ethanedithiol (EDT) Dichloromethane (DCM), *N*,*N*-dimethylformamide (DMF), tert-butyl methyl ether, N,N'-dicycloexylcarbodiimide (DCC), 3-bromopropionic acid, HPLC grade water and acetonitrile were obtained from Sigma Aldrich (St. Louis, MO). 1-Hydroxybenzotriazole hydrate (HOBT) and O-(Benzotriazol-1-yl)-*N*,*N*,*N*',*N*'-tetramethyluronium hexafluorophosphate (HBTU) were obtained from AnaSpec, Inc. (Fremont, CA). Rink-amide-MBHA resin was obtained from Novabiochem (San Diego, CA).

### Synthesis

The peptides were synthesized according to the standard conventional room temperature 9-fluorenylmethoxycarbonyl (Fmoc) chemistry using the Biotage Syro Wave instrument or Syro I MultiSynThec GmbH (Wullener, Germany) automatic synthesizer, on different resins according to the C-terminus to obtain (Rink Amide MBHA resin with 0.38 mmol/g substitution or Wang resin with 1.0 mmol/g), on a scale of 50 µmol. Fmoc deprotection with 30% piperidine in DMF was performed in two stages with an initial deprotection of 5 min followed by 10 min at 25°C.

Solutions of Amino acids, HOBT/HBTU, and DIEA were prepared at the concentration of 0.5M, 0.45M, and 1M in DMF, respectively.

Coupling reactions were performed with a 5 fold excess of Fmoc-AA-OH with 1:1:2 AA/HOBT-HBTU/DIPEA for 30 min at 25°C. Double coupling at RT (each of 30 min) was carried out for the hindered Fmoc-Arg(Pbf)-OH. Peptide capping, at the end of each synthesis, was performed with a 10% solution of acetic anhydride and 6% DIPEA in DMF (double acetylation, each of 20 min). The symmetric anhydride of 3-bromopropionic acid was prepared using 10 eq./resin loading of 3-bromopropionic acid and 5 eq./resin loading of DCC in DCM. For coupling, 3-bromopropionic anhydride was added to the resin in presence of a catalytic amount of DIPEA in DMF, for 1 hour at 25°C.

Cleavage was performed using 94.5:2.5:1:2.5 TFA/H₂O/TIS/EDT for 3 hours at 25°C. Following cleavage the peptide was precipitated and washed in tert-butyl methyl ether.

Disulphide or thioether bond formation was performed by dissolving 1mg/ml of the crude peptide in a 0.1M solution of ammonium bicarbonate, leaving the mixture under stirring in an open vessel for 24 hours. Reactions were monitored by LC/MS.

**LC/MS analysis of peptides.** Peptide purification and LC/MS analysis were performed using a combined system HPLC (Gilson)/ Flexar SQ 300 mass spectrometer with electrospray ionization (Perkin Elmer) or a ESI LC-MS (Thermo Electron Finnigan Surveyor MSQ single quadrupole). Analytical reverse phase-high pressure liquid chromatography (RP-HPLC) was performed using a Jupiter Proteo 10µm C18 250x4.6 mm column, monitoring at 220 and 280 nm with a flow of 1.3 mL/min using as solvent system H₂O/0.1% TFA (A) and CH₃CN/0.1% TFA (B) with a linear gradient 5 - 60% B over 30 min. Peptide purification was performed by semi-preparative reverse phase-high pressure liquid chromatography (RP-HPLC). Semi-preparative chromatography made use of a Jupiter Proteo 90 Å C18 column (10x250 mm, 10 µm) (Phenomenex) and a linear gradient 5 - 60% B over 25 min, flow rate 10 mL/min. The identity of purified peptides was confirmed by electron spray ionization liquid chromatography-mass spectrometry (ESI LC-MS). MS scan was performed in the mass range 200 to 3000 m/z.

### Example 2: Antibacterial activity

### Antibacterial activity assay

A CFU assay of the antibacterial activity of the peptides of the present invention against Pseudomonas aeruginosa ATCC (American Type Culture Collection, Manassas, VA, USA) 27853, Enterococcus faecalis ATCC 29212 and Escherichia coli ATCC 25922 was performed as follows. The strains were grown in aerobic conditions in tryptic soy broth (Difco Laboratories, Detroit, MI, USA) at 37°C and incubated in the presence of hBDs for 2 h at 37°C. We used two concentrations of peptides, i.e., 2.5 µM and 12.5 µM. For salt-dependence assay, 0, 50, 100 and 200 mM NaCl were included in the incubation buffer as previously described (1). Each assay was performed in triplicate. Bactericidal activity (mean and SD of three assays) is expressed as the ratio between colonies counted and the number of colonies on a control plate. The minimal inhibitory concentration (MIC) of the peptide SEQ ID No. 25 was determined with a modified version of the microbroth dilution assay of the National Committee for Clinical Laboratory Standards using a final inoculum of 105 CFU/ml. The following peptide concentrations were used: 100.0, 50.0, 25.0, 12.5, 6.25, 3.12 and 1.56 µM.

The growth performance of Pseudomonas aeruginosa ATCC (American Type Culture Collection, Manassas, VA, USA) 27853, Enterococcus faecalis ATCC 29212 and Escherichia coli ATCC 25922 was also tested.

### Results

The antibacterial activity of SEQ ID No.25 was evaluated in comparison with hBD1 and hBD3 as reference peptides. Two concentrations of each peptide (2.5 and 12.5 µM), and four concentrations of NaCl (0, 50, 100 and 200 mM) were tested against *P. aeruginosa, E. coli* and *E. faecalis,* and the results are reported in Table 1. SEQ ID No.25, tested against *P. aeruginosa* at 2.5 µM exerted similar antibacterial activity compared to hBD3 at 200 mM NaCl; at 12.5 µM, SEQ ID No.25 was more active at 200 mM NaCl.

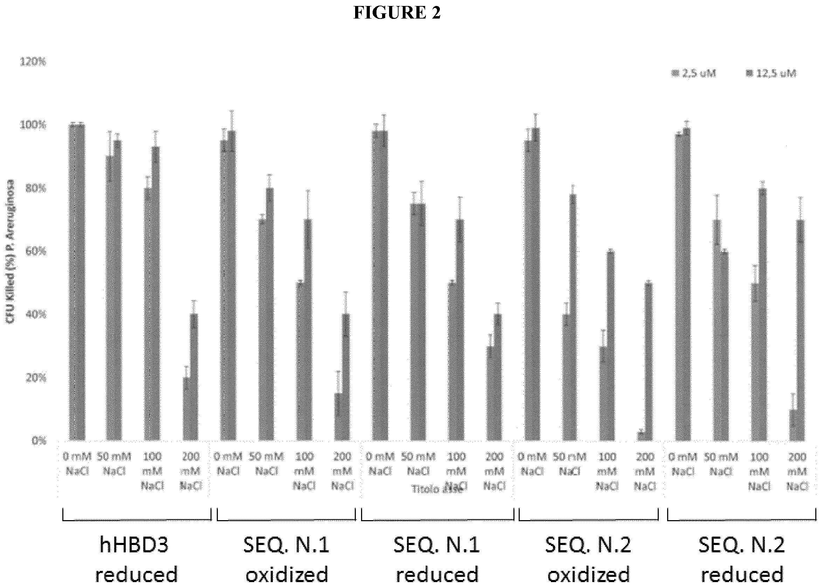

The antibacterial activity of SEQ ID No.25 versus *E. coli* and *E. faecalis* was similar to hBD3 up to 100 mM salt at both 2.5 and 12.5 µM peptide. SEQ ID No. 25 exerted the best antibacterial activity at 12.5 µM: it was more effective than hBD3 in the presence of the highest salt concentrations (200 mM) (Fig. 2B, C). Its MIC ranged between 12.5-25.0 µM for the tested microorganisms. hBD1, hBD3 and SEQ ID No.25 exerted a strong antibacterial effect against *P. aeruginosa, E. coli* and *E*. *faecalis* (12.5 µM).

The reported results show that SEQ ID No. 25 has enhanced antibacterial activity compared to the wild-type hBDs.

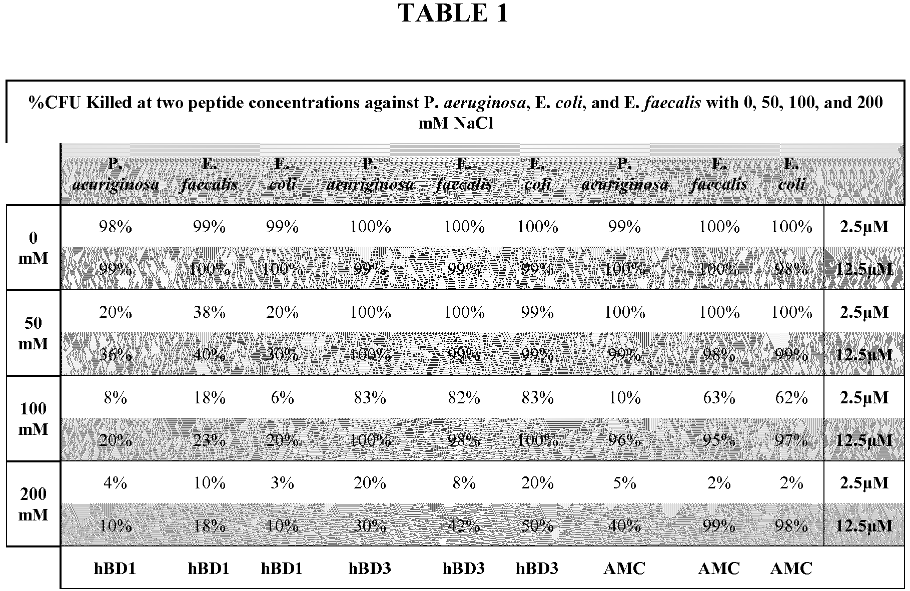

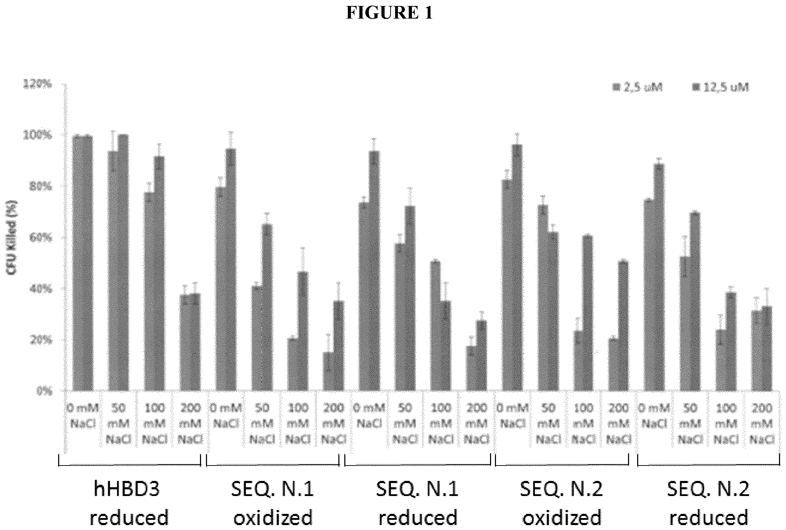

The same method was applied to test the antibacterial activity of SEQ ID No.1 and SEQ ID No.2, in oxidized and reduced form, using hBD3 as reference peptide. Two concentrations of each peptide (2.5 and 12.5 µM), and four concentrations of NaCl (0, 50, 100 and 200 mM) were tested against *P. aeruginosa,* and *E. coli.* The results are reported in Fig. 1 (Antibacterial activity of SEQ. N. 1 and 2 on *E. coli*) and Fig. 2 (Antibacterial activity of SEQ. N. 1 and 2 on *P. aeruginosa).*

### Example 3: Antiviral activity

### Antiviral activity assay

Vero cells were grown in Dulbecco's modified Eagle's medium (DMEM) supplemented with 10% fetal calf serum. HSV-1 carrying a lacZ gene driven by the cytomegalovirus (CMV) IE-1 promoter to express beta-galactosidase was propagated as described elsewhere (Galdiero S, Falanga A, Vitiello M, D'Isanto M, Cantisani M, Kampanaraki A, Benedetti E, Browne H, Galdiero M. Peptides. 2008 Sep;29(9):1461-71). All experiments were conducted in parallel with no-peptide controls. The effect of peptides on inhibition of HSV infectivity on cell monolayers (Tarallo R, Carberry TP, Falanga A, Vitiello M, Galdiero S, Galdiero M, Week M., Int J Nanomedicine. 2013;8:521-34.) was assessed as follows: i) For "co-exposure" experiments, the cells were incubated with increasing concentrations of the peptides (1, 5, 10.0, 20.0, 50.0, 100.0 and 150.0 µM) and with the viral inoculum for 45 min at 37°C. Non-penetrated viruses were inactivated by citrate buffer at pH 3.0. Monolayers were fixed, stained with 5-bromo-4-chloro-3-indolyl-beta-D-galactopyranoside (X-gal) and plaque numbers were scored. Experiments were performed in triplicate and the percentage of inhibition was calculated with respect to no-peptide control experiments; ii) For "virus pre-exposure" experiments, approximately 2 x 10⁴ PFU of HSV-1 were incubated with 20 µM of peptides for 45 min at 37°C then titrated on Vero cell monolayers; iii) For "cell pre-exposure" experiments, Vero cells were incubated with 20 µM of peptides for 30 min at 4°C and infected with serial dilutions of HSV-1 for 45 min at 37°C; and iv) For "post-exposure" experiments, Vero cell monolayers were incubated with virus for 45 minutes at 37°C and afterwards the peptide was added to the inoculum followed by an additional incubation period of 30 minutes at 37°C.

### Results

The antiviral activity of SEQ ID No.25 in both oxidized and reduced form, was evaluated and compared to the activity of hBD3, and the results are reported in Table 2.

The antiviral activity was evaluated in co-exposure experiments (with cell, virus, and peptide at zero time), virus pre-exposure experiments (virus plus peptide before cell infection), cell pre-exposure experiments (cell plus peptide before virus infection) and post-treatment (cell plus virus before peptide addition) in order to evaluate in which phase, if any, the peptides could inhibit virus infectivity. SEQ ID No.25 in its reduced form was always less active than the oxidized version, while it is reported that hBD3 oxidized and reduced showed the same antiviral activity. Both in the co-exposure and virus pre-treatment experiments, the oxidized SEQ. ID.No 25 was able to reduce virus infectivity of 60% at 150 µM, while a reduction of infectivity of 30% was obtained in the cell-pre-treatment experiment, and no effect was seen in the post-treatment experiment. These results indicate that the new peptide is able to interfere with the viral attachment and entry. The oxidized SEQ.ID.No. 25 showed an IC50 of 100 µM, compared to an IC50 of 10 µM for hBD3.

**TABLE 2**

| **Peptide inhibition at different concentration and treatments** | | | | | | | | |
|---|---|---|---|---|---|---|---|---|
| | **1 uM** | **5 uM** | **10 uM** | **20 uM** | **50 uM** | **1000 uM** | **150 uM** | |
| **hBD3** | 79 | 60 | 43 | 32 | 17.5 | 9 | 6.5 | **Co-treatment at 37°C** |
| | 64 | 52 | 33 | 31 | 20 | 10 | 8.5 | **Virus Pre-treatment at 37°C** |
| | 53.5 | 41 | 27 | 22 | 18 | 5 | 1 | **Cell Pre-treatment at 4°C** |
| **SEQ.ID. No 25 ox** | 81 | 76 | 74 | 62 | 60 | 43 | 40 | **Co-treatment at 37°C** |
| | 100 | 72 | 58 | 45 | 44 | 42 | 41 | **Virus Pre-treatment at 37°C** |
| | 100 | 76 | 74 | 71 | 69 | 67 | 65 | **Cell Pre-treatment at 4°C** |
| **SEQ.ID. No 25 red** | 99 | 98 | 95 | 79 | 77 | 67 | 60 | **Co-treatment at 37°C** |
| | 99 | 98 | 95 | 91 | 80 | 77 | 70 | **Virus Pre-treatment at 37°C** |
| | 99 | 98 | 95 | 91 | 89 | 87 | 85 | **Cell Pre-treatment at 4°C** |

The same method was applied to test the antiviral activity of SEQ ID No.1 at different concentrations (1, 5, 10.0, 20.0, 50.0, 100.0 and 150.0 µM). The results are reported in Table 3.

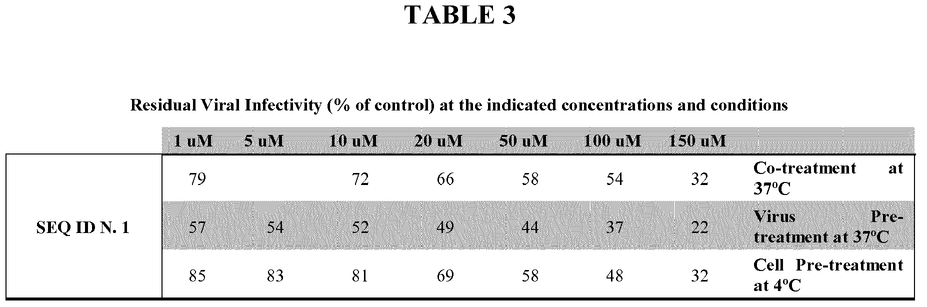

### Example 4: Serum Stability of SEQ ID No.1

The serum stability of SEQ ID No. 1 was assessed as reported in Antcheva N, Morgera F, Creatti L, Vaccari L, Pag U, Pacor S, Shai Y, Sahl HG, Tossi A. 2009. Artificial beta-defensin based on a minimal defensin template. Biochem. J. 421:435-447. Aliquots of SEQ ID No. 1 were incubated with 20% (v/v) human serum at 0, 0.5, 1, 2, 3, 6, 8, and 24 h, and then analysed by LC/MS for peptide integrity. LC/MS was performed on a combined system constituted by a reverse-phase high-performance liquid chromatography (Gilson, Middleton, USA) interfaced with a Flexar SQ 300 mass spectrometer with electrospray ionization (Perkin Elmer, CA, USA). Components were separated using a reverse phase Jupiter C₁₈ analytical column (5-µm particle size, 300-Å pore size, 4.6x250 mm) (Phenomenex, Aschaffenburg, Germany) with a linear gradient of 5 to 60% acetonitrile in acidified water (0.1% trifluoroacetic acid [TFA]) over 30 min. Peptide cleavage in serum was assessed by electron spray ionization mass spectrometry (ESI -MS) analysis. MS scan was performed in the mass range 200 to 3000 m/z.

The results are reported in Table 4. SEQ ID No. 1 is completely stable up to 3h of incubation, and intact peptide is still present after 24 h. Peptide hydrolysis proceeds from the C terminus, with progressive cleavage up to the first cysteine residue. The sequence delimited by the disulfide bridge remains stable thereafter, and represents the only product of degradation identified together with the intact peptide at the end of the incubation period.

**TABLE 4**

| Time-course of the incubation of SEQ ID No. 1 in human plasma | | | | |
|---|---|---|---|---|
| **Time (h)** | **Peptide sequence** | **Theor. Mass** | **Exp. Mass (m/z)** | **Charge state** |
| 0 | Ac-CLPKEEQIGKSSTRGRKSCRRKK-NH₂ | 2715.24 | 1357.79 | +2 |
| | | | 906.05 | +3 |
| | | | 680.10 | +4 |
| 0.5 | Ac-CLPKEEQIGKSSTRGRKSCRRKK-NH₂ | 2715.24 | 1358.30 | +2 |
| | | | 906.07 | +3 |
| | | | 679.70 | +4 |
| 1 | Ac-CLPKEEQIGKSSTRGRKSCRRKK-NH₂ | 2715.24 | 1358.58 | +2 |
| | | | 906.02 | +3 |
| | | | 679.81 | +4 |
| 3 | Ac-CLPKEEQIGKSSTRGRKSCRRKK-NH₂ | 2715.24 | 1358.36 | +2 |
| | | | 906.13 | +3 |
| | | | 679.81 | +4 |
| 6 | Ac-CLPKEEQIGKSSTRGRKSCRRKK-NH₂ | 2715.24 | 1358.58 | +2 |
| | | | 906.02 | +3 |
| | | | 679.71 | +4 |
| | Ac-CLPKEEQIGKSSTRGRKSCRRK-OH | 2587.05 | 1294.95 | +2 |
| | | | 863.21 | +3 |
| | | | 647.31 | +4 |
| | Ac-CLPKEEQIGKSSTRGRKSCR-OH | 2303.69 | 1152.77 | +2 |
| | | | 768.92 | +3 |
| | | | 576.84 | +4 |
| | Ac-CLPKEEQIGKSSTRGRKSC-OH | 2147.5 | 1074.60 | +2 |
| | | | 716.84 | +3 |
| | | | 537.75 | +4 |
| 8 | Ac-CLPKEEQIGKSSTRGRKSCRRKK-NH₂ | 2715.24 | 1357.91 | +2 |
| | | | 905.74 | +3 |
| | | | 679.70 | +4 |
| | Ac-CLPKEEQIGKSSTRGRKSCRRK-OH | 2587.05 | 1294.70 | +2 |
| | | | 862.90 | +3 |
| | | | 647.44 | +4 |
| | Ac-CLPKEEQIGKSSTRGRKSCR-OH | 2303.69 | 1152.55 | +2 |
| | | | 768.70 | +3 |
| | | | 576.70 | +4 |
| | Ac-CLPKEEQIGKSSTRGRKSC-OH | 2147.5 | 1074.13 | +2 |
| | | | 716.65 | +3 |
| | | | 537.47 | +4 |
| 24 | Ac-CLPKEEQIGKSSTRGRKSCRRKK-NH₂ | 2715.24 | 1358.17 | +2 |
| | | | 905.96 | +3 |
| | | | 679.68 | +4 |
| | Ac-CLPKEEQIGKSSTRGRKSC-OH | 2147.5 | 1074.32 | +2 |
| | | | 716.90 | +3 |
| | | | 537.66 | +4 |

## Claims

1. A cyclic peptide selected from the group consisting of SEQ. ID. No. 1, SEQ. ID. No. 2, SEQ. ID. No. 25.

2. A cyclic peptide according to claim 1 in lipidated or PEGylated form.

3. A cyclic peptide according to claim 1 or 2 for use as a medicament.

4. A cyclic peptide according to claim 1 or 2 for use in the treatment of bacterial or viral infections.

5. A pharmaceutical composition comprising a therapeutically-effective amount of a cyclic peptide according to claim 1 and at least one pharmaceutically acceptable excipient and/or carrier.

## Patentansprüche

1. Zyklisches Peptid, ausgewählt aus der Gruppe, bestehend aus SEQ ID NO: 1, SEQ ID NO: 2, SEQ ID NO: 25.

2. Zyklisches Peptid gemäß Anspruch 1 in lipidierter oder PEGylierter Form.

3. Zyklisches Peptid gemäß Anspruch 1 oder 2 zur Verwendung als ein Medikament.

4. Zyklisches Peptid gemäß Anspruch 1 oder 2 zur Verwendung in der Behandlung von bakteriellen oder viralen Infektionen.

5. Pharmazeutische Zusammensetzung, umfassend eine therapeutisch wirksame Menge an zyklischem Peptid gemäß Anspruch 1 und wenigstens einen pharmazeutisch annehmbaren Exzipienten und/oder Träger.

## Revendications

1. Peptide cyclique choisi dans le groupe constitué par SEQ ID n° 1, SEQ ID n° 2, SEQ ID n° 25.

2. Peptide cyclique selon la revendication 1 sous forme lipidée ou pégylée.

3. Peptide cyclique selon la revendication 1 ou 2 pour une utilisation comme médicament.

4. Peptide cyclique selon la revendication 1 ou 2 pour une utilisation dans le traitement d'infections bactériennes ou virales.

5. Composition pharmaceutique comprenant une quantité thérapeutiquement efficace d'un peptide cyclique selon la revendication 1 et d'au moins un excipient et/ou véhicule pharmaceutiquement acceptable.


### SEQUENCE LISTING

<110> CEINGE
<120> Cyclic beta defensins analogs
<130> 2951EUR
<160> 25
<170> PatentIn version 3.5
<210> 1
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<400> 1
   Cys Leu Pro Lys Glu Glu Gln Ile Gly Lys Ser Ser Thr Arg Gly Arg
   1 5 10 15
   Lys Ser Cys Arg Arg Lys Lys
   20
<210> 2
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<400> 2
   Cys Leu Pro Lys Gln Gln Gln Ile Gly Lys Ser Ser Thr Arg Gly Arg
   1 5 10 15
   Lys Ser Cys Arg Arg Lys Lys
   20
<210> 3
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 3
   Xaa Leu Pro Lys Glu Glu Gln Ile Gly Lys Ser Ser Thr Arg Gly Arg
   1               5                   10                  15
   Lys Ser Cys Arg Arg Lys Lys
               20
<210> 4
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 4
   Xaa Leu Pro Lys Gln Gln Gln Ile Gly Lys Ser Ser Thr Arg Gly Arg
   1               5                   10                  15
   Lys Ser Cys Arg Arg Lys Lys
               20
<210> 5
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<400> 5
   Cys Pro Arg Arg Tyr Lys Gln Ile Gly Thr Ser Ser Thr Arg Gly Arg
   1 5 10 15
   Lys Ser Cys Arg Arg Lys Lys
   20
<210> 6
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 6
   Xaa Pro Arg Arg Tyr Lys Gln Ile Gly Thr Ser Ser Thr Arg Gly Arg
   1 5 10 15
   Lys Ser Cys Arg Arg Lys Lys
   20
<210> 7
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<400> 7
   Cys Pro Ile Phe Thr Lys Ile Gln Gly Thr Ser Ser Thr Arg Gly Arg
   1 5 10 15
   Lys Ser Cys Arg Arg Lys Lys
   20
<210> 8
   <211> 23
   <212> PRT
   <213> Synthetic Sequence
<220>
   <221> MISC_FEATURE
   <222> (1)..(1)
   <223> -CH2-CH2-CH2-CO-
<220>
   <221> DISULFID
   <222> (1)..(19)
<400> 8
   Xaa Pro Ile Phe Thr Lys Ile Gln Gly Thr Ser Ser Thr Arg Gly Arg
   1               5                   10                  15
   Lys Ser Cys Arg Arg Lys Lys
               20
<210> 9
   <211> 23
   <212> **PRT**
   <213> Synthetic Sequence
<220>
   <221> MOD_RES
   <222> (1)..(1)
   <223> ACETYLATION
<220>
   <221> DISULFID
   <222> (1)..(1)
<400> 9
   Cys Leu Pro Lys Glu Glu Gln Ile Gly Lys Ser Gly Leu Pro Gly Thr
   1               5                   10                  15
   Lys Ser Cys Arg Arg Lys Lys
               20
<210> 10
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 10
   Xaa Leu Pro Lys Glu Glu Gln Ile Gly Lys Ser Gly Leu Pro Gly Thr
   1               5                   10                  15
   Lys Ser Cys Arg Arg Lys Lys
               20
<210> 11
   <211> 23
   <212> PRT
   <213> Synthetic Sequence
<220>
   <221> DISULFID
   <222> (1)..(19)
<220>
   <221> MOD_RES
   <222> (1)..(1)
   <223> ACETYLATION
<400> 11
   Cys Leu Pro Lys Glu Glu Gln Ile Gly Lys Ser Tyr Arg Gly Lys Ala
   1               5                   10                  15
   Lys Ser Cys Arg Arg Lys Lys
               20
<210> 12
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 12
   Xaa Leu Pro Lys Glu Glu Gln Ile Gly Lys Ser Tyr Arg Gly Lys Ala
   1               5                   10                  15
   Lys Ser Cys Arg Arg Lys Lys
               20
<210> 13
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 13
   Cys Leu Pro Lys Glu Glu Gln Ile Gly Lys Ser Ser Thr Arg Gly Arg
   1               5                   10                  15
   Lys Ser Cys Arg Arg Lys Lys
               20
<210> 14
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 14
   Cys Leu Pro Lys Gln Gln Gln Ile Gly Lys Ser Ser Thr Arg Gly Arg
   1               5                   10                  15
   Lys Ser Cys Arg Arg Lys Lys
               20
<210> 15
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 15
   Xaa Leu Pro Lys Glu Glu Gln Ile Gly Lys Ser Ser Thr Arg Gly Arg
   1 5 10 15
   Lys Ser Cys Arg Arg Lys Lys
   20
<210> 16
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 16
   Xaa Leu Pro Lys Gln Gln Gln Ile Gly Lys Ser Ser Thr Arg Gly Arg
   1 5 10 15
   Lys Ser Cys Arg Arg Lys Lys
   20
<210> 17
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 17
   Cys Pro Arg Arg Tyr Lys Gln Ile Gly Thr Ser Ser Thr Arg Gly Arg
   1               5                   10                  15
   Lys Ser Cys Arg Arg Lys Lys
               20
<210> 18
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<400> 18
   Xaa Pro Arg Arg Tyr Lys Gln Ile Gly Thr Ser Ser Thr Arg Gly Arg
   1               5                   10                  15
   Lys Ser Cys Arg Arg Lys Lys
               20
<210> 19
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 19
   Cys Pro Ile Phe Thr Lys Ile Gln Gly Thr Ser Ser Thr Arg Gly Arg
   1               5                   10                  15
   Lys Ser Cys Arg Arg Lys Lys
               20
<210> 20
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 20
   Xaa Pro Ile Phe Thr Lys Ile Gln Gly Thr Ser Ser Thr Arg Gly Arg
   1               5                   10                  15
   Lys Ser Cys Arg Arg Lys Lys
               20
<210> 21
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 21
   Cys Leu Pro Lys Glu Glu Gln Ile Gly Lys Ser Gly Leu Pro Gly Thr
   1               5                   10                  15
   Lys Ser Cys Arg Arg Lys Lys
               20
<210> 22
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 22
   Xaa Leu Pro Lys Glu Glu Gln Ile Gly Lys Ser Gly Leu Pro Gly Thr
   1               5                   10                  15
   Lys Ser Cys Arg Arg Lys Lys
               20
<210> 23
   <211> 23
   <212> PRT
   <213> Synthetic Sequence
<220>
   <221> MOD_RES
   <222> (1)..(1)
   <223> ACETYLATION
<220>
   <221> DISULFID
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 23
   Cys Leu Pro Lys Glu Glu Gln Ile Gly Lys Ser Tyr Arg Gly Lys Ala
   1               5                   10                  15
   Lys Ser Cys Arg Arg Lys Lys
               20
<210> 24
   <211> 23
   <212> PRT
   <213> Synthetic Sequence
<220>
   <221> MISC_FEATURE
   <222> (1)..(1)
   <223> -CH2-CH2-CH2-CO-
<220>
   <221> THIOETH
   <222> (1)..(19)
<220>
   <221> MISC_FEATURE
   <222> (20)..(20)
   <223> D-Arg
<220>
   <221> MISC_FEATURE
   <222> (21)..(21)
   <223> D-Arg
<220>
   <221> MISC_FEATURE
   <222> (22)..(22)
   <223> D-Lys
<220>
   <221> MISC_FEATURE
   <222> (23)..(23)
   <223> D-Lys
<400> 24
   Xaa Leu Pro Lys Glu Glu Gln Ile Gly Lys Ser Tyr Arg Gly Lys Ala
   1               5                   10                  15
   Lys Ser Cys Arg Arg Lys Lys
               20
<210> 25
   <211> 17
   <212> PRT
   <213> Synthetic Sequence
<220>
   <221> DISULFID
   <222> (1)..(11)
<400> 25
   Cys Pro Ile Phe Thr Lys Ile Gln Gly Thr Cys Gly Gly Arg Arg Lys
   1               5                   10                  15
   Lys